(19) Europäisches Patentamt / European Patent Office / Office européen des brevets

(11) **EP 2 091 415 B1**

(12) **EUROPEAN PATENT SPECIFICATION**

(45) Date of publication and mention
of the grant of the patent:
**08.07.2015 Bulletin 2015/28**

(51) Int Cl.:
*A61B 5/00* *(2006.01)*    *A61B 5/107* *(2006.01)*
*G01B 11/06* *(2006.01)*

(21) Application number: **07819728.2**

(22) Date of filing: **09.11.2007**

(86) International application number:
**PCT/EP2007/009735**

(87) International publication number:
**WO 2008/058682 (22.05.2008 Gazette 2008/21)**

(54) **DETERMINING A THICKNESS OF A LAYER OF FAT OF AN ORGANISM**

BESTIMMUNG EINER DICKE EINER FETTSCHICHT EINES ORGANISMUS

DÉTERMINATION DE L'ÉPAISSEUR D'UNE COUCHE DE GRAISSE D'UN ORGANISME

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **14.11.2006 EP 06023691
14.11.2006 US 865738 P**

(43) Date of publication of application:
**26.08.2009 Bulletin 2009/35**

(73) Proprietor: **Medizinische Universität Graz
8036 Graz (AT)**

(72) Inventor: **MÖLLER, Reinhard
8010 Graz (AT)**

(74) Representative: **Dilg, Haeusler, Schindelmann
Patentanwaltsgesellschaft mbH
Leonrodstrasse 58
80636 München (DE)**

(56) References cited:
**EP-A- 0 516 251        WO-A1-95/11460
US-A- 5 014 713        US-A1- 2004 193 025
US-A1- 2005 043 598        US-A1- 2006 041 198
US-B1- 6 591 125**

## Description

The invention relates to an apparatus for determining a thickness of a layer of fat of an organism.

The invention further relates to a method of determining a thickness of a layer of fat of an organism.

Moreover, the invention relates to a program element.

Further, the invention relates to a computer-readable medium.

For medical applications, it may be desirable to measure a thickness of a layer of fat at a specific body part of a human being.

US 5,014,713 discloses a body fat thickness measuring device comprising a pair of infrared emitting diodes, one emitting a steady, low-intensity light, and the other emitting periodic, high-intensity pulses of light, to be placed against the skin where the fat is to be measured. The device contains an array of detectors in the form of infrared-sensitive photo-transistors which are placed against the skin in predetermined locations near the two diodes, yet shielded from ambient light. The detector array provides signals proportional to the amount of infrared light detected, and these signals are summed and amplified, forming a composite signal. The amplitude of this composite signal, which is indicative of the thickness of the layer of fat, is displayed on a digital readout device.

US 2007/185399 discloses a portable body fat measurement apparatus using a near infrared ray, the apparatus including a near infrared sensor to receive a second near infrared ray reflected from a body part of a user after the body part is irradiated with a first near infrared ray, and to convert the second near infrared ray into an electrical signal, an alternating current signal extraction unit to extract an alternating current component from the electrical signal, and a body fat measurement control unit to compare an amplitude of the alternating current component with a predetermined threshold, and to generate an alarm signal when the amplitude of the alternating current component meets the threshold.

EP 0,516,251 discloses a method and a device for detecting the thickness of a layer of fat, in which the layer of fat is illuminated at different sites. In the process, the light intensity emanating from the layer of fat is measured to determine the illumination at the various sites, and the thickness of the layer of fat is inferred from the relationship of the various intensities.

US 2005/0043598 A1 relates to systems and methods for minimizing or eliminating transient non-glucose related signal noise. The system monitors a data stram from a glucose sensor and detects signal artifacts that have higher amplitude than electronic or diffusion related system noise. Such high amplitude signal artifacts occur in an glucose sensor requiring an enzymatic reaction. Local ischemia creates a reaction that is rate-limited by oxygen, which is responsible for low noise. In this situation glucose would be expected to build up in the membrane because it would not completely catabolized during the oxygen deficit. When oxygen is again in excess, there would also be excess glucose due to the transient oxygen deficit. The enzyme rate would speed up for a short period until excess glucose is catabolized, resulting in high noise. In an acceptance filter, each new sampled data point is compared against the most representative estimateof the sensor curve at the previous sampling interface, or at a projection to a current estimated value. If a ratio of the current value to a comparison value is greater or less than a certain threshold, then the current data point is replaced by a previously accepted value.

AT 201095 discloses a device for diagnosing the state of health of human individuals, in particular for detecting diseases (illnesses, disorders, conditions) with the aid of a unit for surveying specific bodily characteristics, and of a unit for compiling diagnoses therefrom. It is provided that, in order to detect chronic metabolic complaints, the diagnostic system comprises a unit which can be positioned in a defined way relative to body topography, for determining the thickness of the subcutaneous layer of fat, which unit is connected, so that it can transmit signals, to a diagnostic compiling unit which, for its part, has a current-signal storage unit for receiving, collecting, storing and reproducing signals corresponding to data on subcutaneous fat layer thickness, a current-signal unit, connected to the current-signal storage unit, for processing the current signals to form current characteristics of fat layer thickness with the aid of a unit, for the purpose of storing them, a storage unit for health data and comparative data, having characteristics obtained from a first group of test subjects (probands) in good health at precisely defined points in each case, a storage unit for sickness data and comparative data which can hold data on subcutaneous fat layer thickness obtained from a further group of comparative test subjects respectively suffering from one of the chronic metabolic complaints, and has a diagnostic unit which is connected to each of the said units and, for its part, is fitted with a subunit for standardizing the characteristics respectively currently collected, having health characteristics and comparative illness characteristics which can be supplied by the comparative data storage units and are preferably standardized, in accordance in each case with medically relevant criteria, with a comparator subunit, fitted with comparative algorithms, for comparing the standardized current characteristics with health characteristics and comparative illness characteristics, and with a diagnostic output unit connected to the subunit, as well as a test subject/diagnosis storage unit.

However, it may be difficult to determine a thickness of a subcutaneous fat layer based on detected signals. Therefore, there may be a need for an efficient algorithm for determining a thickness of a layer of fat based on signals obtained by an optical measurement.

It is an object of the invention to provide an accurate system of determining a thickness of a fat layer based on detected signals.

In order to achieve the object defined above, an apparatus for determining a thickness of a layer of fat of an organism, a method of determining a thickness of a layer of fat of an organism, a program element, and a computer readable medium according to the independent claims are provided.

According to the invention, an apparatus for determining a thickness of a layer of fat of an organism is provided, as defined in claim 1.

According to another aspect of the invention, a method of determining a thickness of a layer of fat of an organism is provided, as defined in claim 14

According to still another aspect of the invention, a program element (e.g. a software routine, in source code or in executable code) is provided, as defined in claim 15.

According to yet another aspect of the invention, a computer-readable medium (e.g. a CD, a DVD, a USB stick, a floppy disk or a harddisk) is provided, as defined in claim 16.

The lipid layer thickness estimation scheme according to embodiments of the invention can be realized by a computer program, that is by software, or by using one or more special electronic optimization circuits, that is in hardware, or in hybrid form, that is by means of software components and hardware components.

In the context of this application, the term "layer of fat" may particularly denote any layer of a lipid material which is positioned close to a surface of a body of an organism. More particularly, a subcutaneous fat layer may be a fat layer which is located directly below the skin of an organism, but above lower positioned organs like muscles, etc.

The term "organism" may particularly denote any living or dead biological system, particularly a biological system having a metabolism which accumulates fat at specific body positions. Examples for such organisms are human beings, animals, etc.

The term "electromagnetic radiation" may particularly denote a beam of photons of any appropriate wavelength. This may include the optical spectrum (for instance the range between 400 nm and 800 nm), but may also include electromagnetic radiation of other wavelengths, like UV, infrared, or even X-rays. According to exemplary embodiments of the invention, such electromagnetic radiation may be used as a probe, since this electromagnetic radiation is directed through a skin of a body, through the lower laying fat tissue, for reflection/scattering/backscattering at a border between the fatty layer and lower lying layers, for instance muscles.

The term "deviation from a predetermined threshold value" may particularly denote a scheme in which it is analyzed whether a fat layer thickness value deviates from an average value by more than a certain percentage (for instance ±10%) and/or by more than a certain absolute value (for instance ±1mm). Particularly, a logical AND combination of these two criteria may be performed, to obtain a reliable result.

According to an exemplary embodiment of the invention, a system is provided which is capable of accurately measuring a thickness of a fat layer of an organism by using an efficient signal evaluation algorithm. For this purpose, a plurality of light pulses may be emitted by grouped electromagnetic radiation sources (for instance light emitting diodes, LED) to thereby illuminate specific portions of the body with corresponding illumination patterns. For different pulses, different light sources may be active, one or more light sources being activated at a time. Wishing not to be bound to a specific theory, it is presently believed that such light travels through the thin skin of a human being and through the fat layer and may be manipulated (probably reflected) at a border between the fat layer and lower lying tissue, like muscles. However, it may also be possible that a fat tissue structure acts as some kind of optical fiber through which the light is conducted. When travelling through the fat tissue, scatter/backscatter procedures may occur. After reflection/scattering/backscattering, the electromagnetic radiation travels back through the fat layer and the skin and may be detected by one or more electromagnetic radiation detectors, for instance one-pixel detectors (such as photodetectors) or multiple-pixel detectors (such as a CCD camera or a CMOS camera).

It has been recognized by the inventor that the relationship between the individual intensities of the detected light pulses at the site of the detector is an accurate fingerprint of the thickness of the fat layer, even more than absolute values of the signals. Analyzing such ratios between the detection signals may therefore allow to determine the thickness of the fat layer with high accuracy. However, due to a non-homogeneous material structure in the fat layers of organisms, it may happen that individual pulses or ratios do not provide for sufficiently reliable and accurate information regarding the thickness of the fat layer. For this purpose, the thickness of the fat layer is calculated a plurality of times and therefore redundantly based on the different ratios of the signals. When an average value from each of the these individual values has then been calculated, it is verified whether one or more of the calculated individual values deviates more than a threshold value (of, for instance, 10%) from an arithmetic average value or from a median of the individual values. If this is the case (for instance since the corresponding light has propagated through non-homogeneous anatomic parts of the fat layer), then these suspicious measurements are neglected for the final determination of the thickness of the fat layer. In order words, an average thickness is calculated using only the other thickness values without taking into account the strongly different values. Therefore, a high accuracy may be obtained with a numerically simple analysis.

According to an exemplary embodiment of the invention, an optical measurement system (which may be denoted as a "Lipometer") may emit, in a short time, a sequence of geometrically varying light patterns for introduction through the skin into the lower laying fat tissue. Scattered light contributions assigned to these light patterns may be measured by a photodetector. Based on these detection signals, a thickness of a layer of fat tissue may be calculated. The calibration and the evaluation of the system may include a baseline correction to eliminate or suppress artefacts from the detection signals resulting from background radiation, etc. The calibration may also include the comparison with layer thicknesses determined from CT (computed tomography) and/or MRI (Magnetic Resonance Imaging) images as a reference.

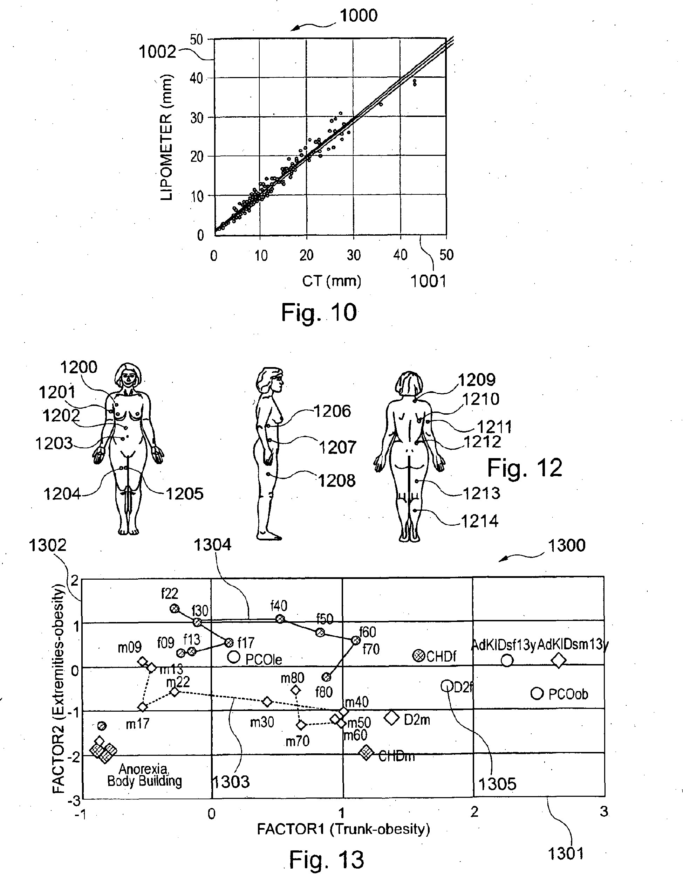

Therefore, the Lipometer may allow for an accurate determination of the individual fat layer thickness at a specific body part of the organism. It is also possible that the fat distribution along the entire body may be analyzed, for instance by measuring at several anatomically standardized measurement positions (for instance 15 measurement positions, see Fig. 12). Therefore, it is possible to obtain more detailed information regarding the individual fat distribution, which may be a result of genetics, lifestyle and/or diseases. Comparing the individual characteristics of the fat layer relationships along the body with reference profiles and databases (of healthy persons and/or of persons having specific diseases like diabetes or coronal diseases) may allow to perform an accurate prediction or diagnosis regarding the risk or presence of specific diseases.

Statistical methods (least square fits, factor and cluster analysis, ROC curves, neural networks, fuzzy logic, etc.) may allow to properly assign risks or the presence of specific diseases simply on the basis of the body fat profiles.

According to an exemplary embodiment of the invention, a simple handheld (for instance wireless) device may be provided which has a measuring head having a measuring surface which is shaped and dimensioned to be brought in direct contact with a body portion to be analyzed. Then, a sequence of light pulses is emitted by the spatially distributed light sources, wherein for each pulse one or a plurality of individual ones of the light sources contribute to the emission of light. The reflected/scattered/backscattered light probe is detected by a photodetector so that the individual intensities of the signals are supplied to a processing or calculating unit, like a CPU, a microprocessor or a computer. On a display (such as an LCD) of this simple embodiment, the result, namely the thickness of the corresponding body portions is then displayed perceivable for a user. A degree of reliability of the measurement may be indicated on the handheld device by means of a multiple colour LED. The colour of the LED(s) may indicate whether the measurement is very reliable (for instance blue colour), sufficiently reliable (for instance green colour), suspicious (for instance yellow colour), or not acceptable (red colour, for instance). The determination of the quality may be performed based on a ratio of used thickness values and estimated thickness values. The described simple embodiment may be appropriate for a personal use, for instance to assist a person being during a diet.

In a more complex embodiment, such a handheld device may be communicatively coupled, for instance via a USB connection or via a wireless data communication path like Bluetooth, to a laptop or other computer so that the results may be supplied from the handheld device to the computer, whereas a software routine running on the powerful computer may perform a more detailed calculation. For instance, the information which body portion has been measured may be supplied to the computer so that a detailed body profile can be derived and compared with database information so that the individual risks for diseases as compared to comparing groups may be displayed in an intuitive manner on the display of the laptop.

For example, it is possible to perform a plurality of (for instance 15) measurements at specific portions of the body. These 15 measurement results may then be condensed to a lower number of factors, for instance to two factors, such as the fat distribution at the trunk and the fat distribution at the extremities of the body. Plotting the results on a two-dimensional diagram may then allow to analyze the individual result on a statistical landscape of diseases which shows that specific diseases like coronal diseases or diabetes are correlated with high accuracy to specific ratios of these two condensed factors. This more complex embodiment may be used for medical purposes or in fitness centres.

In the following, further exemplary embodiments of the apparatus will be explained. However, these embodiments also apply to the method, to the program element and to the computer-readable medium.

The determining unit may be adapted for determining a plurality of values of the thickness of the layer of fat based on a correlation function (phenomenologically) correlating the plurality of ratios between the detected signals with the plurality of values of the thickness of the layer. It has been found to be reasonable to calculate the individual ratios between the detected pulse intensities. For instance, if three pulses a, b, c have been emitted, three ratios a'/b', a'/c', b'/c' of the detected signals a', b', c' may be determined, wherein the detection signal a' is assigned to the exciting signal a, the detection signal b' is assigned to the exciting signal b, and the detection signal c' is assigned to the exciting signal c. The layer thickness determined by each of the ratios may then be formulated as a correlation function, particularly as a polynomial function, more particularly as a polynomial function of the third order or of the fifth order. The coefficients of the individual polynomial factors may then be determined by routine experiments carried out at a sufficiently large number of probands or by a calibration using compare results of another method of determining a thickness of layers, like CT. In other words, alternative methods of measuring fat thickness (CT methods, etc.) may be used for such a correlation. It has been found that already a polynomial of the third order may be sufficient and appropriate to obtain reasonable results.

The determining unit may be adapted for calculating an arithmetic average thickness of the layer of fat. Thus, when the individual values for the layer of thickness have been calculated on the basis of the different ratios of the measurement intensities, an arithmetic average has turned out to yield meaningful results. Alternatively, a median or any other average scheme may be used.

The determining unit may further be adapted for calculating the average thickness of the layer of fat using detection signals measured during the intervals of the (response) pulses and using detection signals in time intervals between subsequent pulses. By taking this measure, it is possible to normalize the useful signals indicative of the emitted pulses having propagated through the fat tissue by subtracting underground values. Such an underground analysis may be particularly precise when not an average baseline value is used for corrected all the pulses, but when for each individual pulse the corresponding previous and subsequent dark phase is used as a basis for performing the underground analysis. Therefore, more particularly the determining unit may be adapted for performing the background correction by subtracting an arithmetic average of detection signals in time intervals before and after a pulse from a detection signal during the pulse, allowing for a more specific correction. Therefore, local artifacts in the signal may be suppressed or eliminated, thereby further increasing the accuracy of the measurement.

The plurality of electromagnetic radiation sources may be adapted for illuminating the layer of fat with a plurality of repetitive (and identical) sequences of the plurality of pulses of electromagnetic radiation. Accordingly, the determining unit may be adapted for summing corresponding detection signals of the plurality of repetitive sequences. For example, 200 measurement sequences with three pulses may be carried out. When one pulse has a length of 50 $\mu$s and a dark phase between two subsequent pulses is 50 $\mu$s, a measurement of 200 repetitive sequences may even be carried out in a reasonable measurement time of less than one second. It is possible that each of the individual sequences is evaluated individually and that resulting thickness values are subsequently averaged over the sequences. It is also possible that the corresponding pulses are summed up over the sequences, as a basis for a single common evaluation of the thickness of the lipid layer.

The apparatus may comprise a quality estimation unit adapted for estimating a quality of the determination of the thickness of the layer of fat based on an amount of values used for calculating the average thickness of the layer of fat. Such a quality estimation unit may simply compare the number of "used" ratios with the entire number of the ratios. If all the ratios can be used since the deviation of the individual fat layer thickness calculation is less than a threshold value of, for instance, 10%, then the measurement can be considered to be very accurate. The more ratios are omitted due to an exceeding deviation from an average value, the lower is the reliability of the measurement which can be reflected in the output of the quality estimation unit. Such an output may be performed optically (for instance using a display), acoustically (for instance using a loudspeaker), or even haptically (for instance using a vibration). An acoustic output, for instance using an LED having different colours with an intuitive colour-quality-assignment may be appropriate.

The apparatus may comprise a user interface adapted for enabling a bidirectional communication between the user and the apparatus. Such a user interface may comprise a display unit like an LCD, a TFT, a plasma device, or even a cathode ray tube. It may also include input elements like a keypad, a joystick, a trackball, or even a microphone of a voice recognition system. It is also possible that individual control buttons are provided on the apparatus. Such a user interface may allow to communicate with a measuring head in a wireless or wired manner. For a wired connection, a USB connection may be used. For a wireless alternative, Bluetooth or infrared communication may be used. It is also possible to transmit results via a data network such as an intranet or the Internet.

The plurality of electromagnetic radiation sources and the at least one electromagnetic radiation detector may be accommodated in a casing in a manner that the plurality of electromagnetic radiation sources and the at least one electromagnetic radiation detector are positionable directly onto the body part of the organism at which the thickness of the layer of fat is to be determined. For this purpose, the measurement head may be shaped in a flattened manner at a measuring portion so that it is possible to directly place it on the skin. The cross-sectional area of this portion should be not too large so as to obtain a high spatial resolution. It is possible that the measurement head has a diameter of 2 cm to 7 cm.

The apparatus may be adapted for determining the thickness of the layer of fat at a plurality of body portions of the organism. For instance, 15 body portions may be analyzed. It may be advantageous that some body portions are defined at the extremities, and some of the body portions are defined at the trunk of the body. This may allow to obtain meaningful results with regard to a correlation of a fat distribution along the body surface with specific diseases like diabetes or coronal diseases.

The apparatus may comprise a diagnosis unit adapted for deriving a medical diagnosis regarding at least one illness based on a relationship of the thickness of the layer of fat at the plurality of body portions of the organism. This may be specifically meaningful if the plurality (for instance 15) of body thicknesses are reduced to, for instance, two groups (extremities and trunk). Then, a fat landscape is obtained on which a specific proband has a specific position. On this landscape, the risk for individual diseases may be plotted as well, based on a statistically or empiric knowledge. Thus, a meaningful diagnosis of risks or already present illnesses may be given.

The number of the plurality of electromagnetic radiation sources illuminating the layer of fat may differ for different ones of the plurality of pulses of electromagnetic radiation, wherein the number may be the larger the larger the distance of the respective electromagnetic radiation sources from the at least one electromagnetic radiation detector is. Since the light scattering depends on the length along which the light beams are traveling, the number of light emitting light sources may be made larger the larger the traveling distance is. This may allow to obtain meaningful intensities of the individual pulses at the detection position of the photodetector.

Additionally or alternatively, an illumination intensity of the plurality of electromagnetic radiation sources illuminating the layer of fat may differ for different ones of the plurality of pulses of electromagnetic radiation, wherein the illumination intensity may be the larger the larger the distance of the respective electromagnetic radiation sources from the at least one electromagnetic radiation detector is. Therefore, it is not necessary to vary the number of active light sources for the different measurement pulses, but it is also possible that different light sources have different illumination intensities. For instance, light sources being located far away from the photodetector may emit a more intense light than those located close to the detector.

The apparatus may be adapted for determining the thickness of the layer of subcutaneous fat of the organism. It has been turned out that neither the genus nor the skin colour of a proband has a significant influence on the accuracy of the layer fat thickness measurement, since the thickness of the skin is usually very small compared to typical fat layer thicknesses. Therefore, the apparatus may be used accurately for determining subcutaneous tissue dimensions.

The apparatus may be adapted as a portable device, particularly as a mobile phone, a personal digital assistant (such as a Palm™), an MP3 player, a gaming device, an audio player, a DVD player, a CD player, a harddisk-based media player, a medical communication system or a body-worn device. Therefore, the Lipometer may be used in many different fields, for instance to provide an additional feature on functional system like a mobile phone. However, it is alternatively possible that the apparatus is used merely for the Lipometer function, if desired.

The apparatus may also be adapted as a measuring head being connectable to a computer, particularly via a USB interface or a wireless interface (like Bluetooth).

The plurality of electromagnetic radiation sources may be adapted for illuminating the layer of fat with the plurality of pulses of electromagnetic radiation in an optical wavelength range (for instance between 400 nm and 800 nm), particularly in a wavelength range between essentially 635 nm and essentially 670 nm, more particularly at a wavelength of about 660 nm. Generally, embodiments of the invention may be implemented with electromagnetic radiation between 350 nm and 2 $\mu$m. However, the present inventor has surprisingly recognized that in contrast to conventional approaches relying on infrared radiation the optical range is particularly appropriate for measuring fat thickness. Although infrared radiation may penetrate deeper in fat tissue than optical light, the combination of the deepness of penetration/extinction into the fat and the forward scattering and backward scattering properties is much better for optical light, particularly between 635 nm and 670 nm (for instance around 660 nm). Thus, the use of optical light may significantly improve the accuracy of the measurement.

In the following, further exemplary embodiments of the method will be explained. However, these embodiments also apply to the apparatus, to the program element and to the computer-readable medium.

The method may comprise providing average thicknesses of the layer of fat measured at a plurality of body portions to a computer-implemented system, and receiving information regarding specific risks for diseases from the computer-implemented system based on a comparison of the provided average thicknesses with values of a number of probands stored on the computer-implemented system. The information may be received from the computer-implemented system via an Internet platform (particularly via the public Internet, the world wide web).

Thus, a user may provide a computer with results of fat thickness measurements performed, for instance, with a handheld apparatus at, for instance, 14 positions of the user's body. For example, a user may access an Internet page and may input the values. If desired, the user may add additional biological information such as age, sex, etc. On the Internet based computer, a database may be present on which statistically significant information taking from a number of probands, for instance from 1000 probands, is stored. Based on a comparison of the database information with the user-specific values, the system may output to the user information indicative of risks for specific diseases, as well as a proposal what to do to reduce such risks. Such a service may be provided against payment by a user. Information which may be output to a user may include a comparison of the values with comparable probands (for instance having a similar age).

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.

Fig. 1 illustrates an apparatus for determining a thickness of a layer of fat of an organism according to an exemplary embodiment of the invention.

Fig. 2A and Fig. 2B show a plan view and a three-dimensional view of a measurement head of an apparatus for determining a thickness of a layer of fat of an organism according to an exemplary embodiment of the invention.

Fig. 3 illustrates a sequence of light pulses emitted by light sources of an apparatus for determining a thickness of a layer of fat of an organism according to an exemplary embodiment of the invention.

Fig. 4 and Fig. 5 show response signals detected by a photodetector of an apparatus for determining a thickness of a layer of fat of an organism according to an exemplary embodiment of the invention in response to the exciting signals of Fig. 3 for the case of a relatively thin layer of fat (Fig. 4) and a relatively thick layer of fat (Fig. 5).

Fig. 6 to Fig. 9 show plan views of measurement heads of apparatuses for determining a thickness of a layer of fat of an organism according to exemplary embodiments of the invention.

Fig. 10 is a diagram illustrating a correspondence of determined fat layer thicknesses of a CT reference method and of a Lipometer according to an exemplary embodiment of the invention.

Fig. 11 shows a protocol of a measurement of a thickness of a layer of fat at various body positions by an apparatus for determining a thickness of a layer of fat of an organism according to an exemplary embodiment of the invention.

Fig. 12 specifies the 15 body positions used for the measurement of Fig. 11.

Fig. 13 shows a landscape of data reduced obesity factors correlated with different diseases according to an exemplary embodiment of the invention.

The illustration in the drawing is schematically. In different drawings, similar or identical elements are provided with the same reference signs.

In the following, referring to **Fig**. 1, an apparatus 100 for determining a thickness of a layer of fat of an organism according to an exemplary embodiment of the invention will be explained.

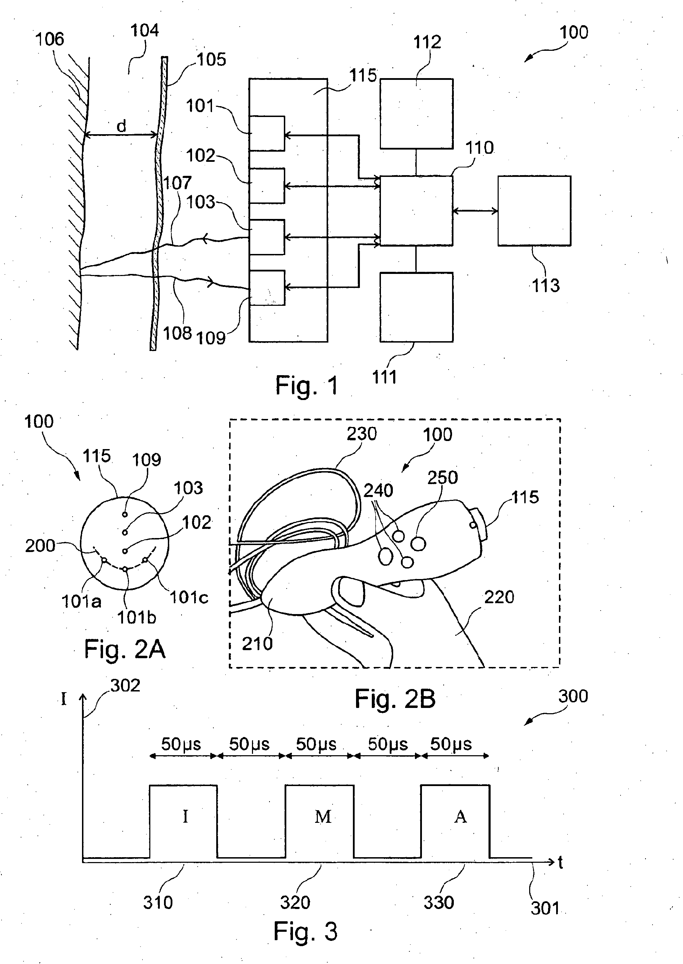

The apparatus 100 comprises a first LED 101 (or an array of LEDs), a second LED 102, and a third LED 103 as a plurality of light sources for illuminating a layer of fat 104 located between a skin 105 and muscle tissue 106 of a human being with a plurality of light pulses 107. As can be taken from Fig. 1, the light pulses 107 may be scattered at the layers 104 to 106 or may be reflected/scattered/backscattered at a border between the fat layer 104 and the muscle layer 106 and are directed towards a photodetector 109 for detecting the detection signals 108 indicative of the light pulses 107 after transmission through the layer of fat 104.

A determining unit 110, for instance a CPU or microprocessor or a computer, may control the emission and the timing of the exciting light pulses 107 of the light sources 101 to 103 and may receive the results from the detector 109. It may store measurement results in a memory device 111, for instance an EEPROM, and may have access to a database 112 at which a plurality of previous measurements at the same human being or from other probands may be stored. The CPU 110 may perform calculations for determining a thickness "d" of the fat layer 104 based on an analysis of a plurality of ratios between the detection signals, as will be described below.

The CPU 110 may be in bidirectional data communication with a user input/output device 113 so that a user may read the measurement result "d" on a display and may also evaluate the quality of the measurement, define measurement conditions, etc.

Fig. 2A and Fig. 2B show more details about the actual construction of the device 100.

**Fig. 2A** shows a plan view of a flat measurement head 115 of the apparatus 100.

As can be taken from Fig. 2A, the light source 103 located closest to the detector 109 and the light source 102 located between the light sources 103 and 101 are realized as individual LEDs. In contrast to this, the LED 101 which is most far away from the detector 109 is realized as three individual LEDs 101a, 101b, 101c arranged on a circle or parabolic trajectory 200. By taking this measure, the light intensities of the remote diodes 101a to 101c may be sufficient to obtain a properly resolved corresponding detection signal so that the detector 109 detects meaningful results from these LEDs 101a to 101c as well.

**Fig. 2B** shows a three-dimensional view of the apparatus 100.

A measurement head is indicated with reference numeral 115. A hand piece 210 can be easily carried by a hand 220 of a human user. Further, a cable connection 230 is shown which allows to connect the apparatus 100 to an analysis computer (not shown).

Operating buttons 240 (such as a power-on button, a measurement start button, etc.) are shown as well by means of which a human user may control the operation of the device 100. Furthermore, an LED 250 as an indicator lamp is shown from which the quality of the measurement can be derived using an intuitive colour scheme.

In the following, referring to Fig. 3 to Fig. 5, the operation and the evaluation of the detection signals to derive a thickness of the fat layer will be described in more detail.

**Fig. 3** shows a diagram 300 having an abscissa 301 along which the time is plotted. Along an ordinate 302, the intensity of the light emitted by the light sources 101 to 103 is shown. A first light pulse 310 indicates a light pulse emitted by the inner light source 103. A second pulse 320 is indicative of an illumination by the middle light source 102, and a third light pulse 330 is indicative of a light pulse emitted by the outer diodes 101a to 101c. Each pulse has a length of 50 μs, and two subsequent pulses are separated by a dark phase of 50 μs, respectively.

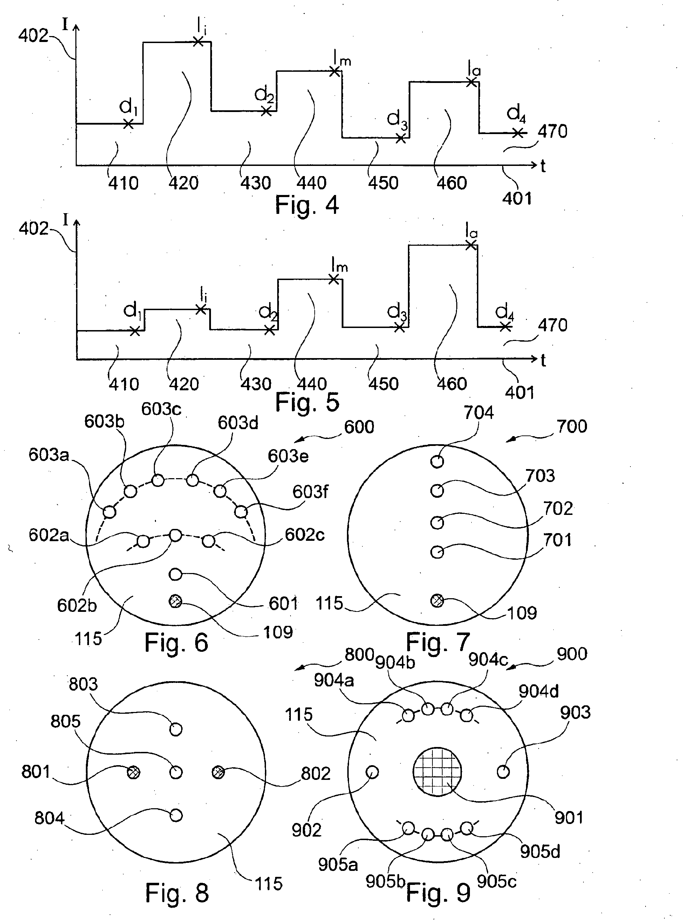

**Fig. 4** shows a diagram 400.

Along an abscissa 401 of the diagram 400, the time is plotted. Along an ordinate 402, the intensity of the signals detected by the photodetector 109 is plotted. Fig. 4 shows a first dark phase 410, a first detection phase 420, a second dark phase 430, a second detection phase 440, a third dark phase 450, a third detection phase 460 and a fifth dark phase 470. At measurement points denoted with small crosses in Fig. 4, four dark phase signals d1, d2, d3 and d4 are measured. Furthermore, three detection pulses li, lm and la are detected. These signals may be converted by an analog-to-digital converter (ADC) into digital signals. As can be taken from Fig. 4, even in the dark phases 410, 430, 450, 470, some light intensity is measured due to background illumination, etc.

Fig. 4 corresponds to a measurement result which is obtained when the layer of fat 104 is relatively thin. In the case of a relatively thick layer thickness, the diagram 500 shown in **Fig. 5** is obtained. As can be taken from a comparison of Fig. 4 and Fig. 5, the respective intensity ratios of the individual signals 420, 440, 460 is a fingerprint of the thickness of the layer of fat.

In the following, it will be explained how the value of the layer thickness is determined based on the measurement intensities d1 to d4, li, lm and la.

The evaluation may suffer from the problem that the light 107, 108 has to propagate the skin 105 twice. Therefore, the spectroscopic result may be dependent on the thickness of the skin 105, from the light conditions, from the skin colour, from the chemical composition of the skin 105 material, etc. In this respect, the inventor has recognized that it may be highly advantageous to evaluate not the absolute light intensity values li, la, lm, but to use ratios. By considering ratios between li, lm and la taking into account also the signals d1 to d4, parameters like skin colour, skin thickness, and other disturbing and varying parameters may be eliminated mathematically.

As can be taken from Fig. 4 and Fig. 5, seven measurement signals d1 to d4, li, lm, la are obtained. However, since a biologically active system (namely a human being) is used as the "measurement sample", an individual detection pulse sequence as shown in Fig. 4 or Fig. 5 may be dependent on actual biological conditions, like breathing conditions, a pulse state, etc. To eliminate such effects by averaging over time, it may be advantageous to repeat the measurement of Fig. 4 or Fig. 5 a plurality of times, for instance N=200 times. Then, each of the individual signals d1 to d4, li, lm and la may be accumulated so that seven sum signals D1 to D4, LI, LM and LA are obtained.

$$D1 = \sum_N d1$$

$$D2 = \sum_N d2$$

$$D3 = \sum_N d3$$

$$D4 = \sum_N d4$$

$$LI = \sum_N li$$

$$LM = \sum_N lm$$

$$LA = \sum_N la$$

It is also possible that each of the seven sum signals D1 to D4, LI, LM and LA is divided by N=200 before further analysis.

Alternatively, the 200 measurements may be evaluated 200 times individually, and the resulting layer thicknesses may be then averaged.

Next, an underground correction may be carried out based on the sum signals D1 to D4, LI, LM and LA:

Corrected pulse intensities LIC, LMC, LAC are obtained by the following equations:

$$LIC = LI - (D1+D2)/2$$

$$LMC = LM - (D2+D3)/2$$

$$LAC = LA - (D3+D4)/2.$$

By taking this measure, influences such as underground, noise, scattered light, and disturbing light may be removed from the measurement.

Thus, the three underground corrected signals LIC, LMC, LAC are obtained.

Next, ratios between these signals are calculated:

$$W1=LMC/LIC$$

$$W2=LAC/LMC$$

$$W3=LAC/LIC$$

Then, a layer thickness may be calculated three times based on the determined ratios, using the following equations. The individual values of the individually calculated layer thicknesses are denoted as MI, AM, AI:

$$MI=\alpha1+\beta1 W1+\gamma1 W1^2+\delta1 W1^3$$

$$AM=\alpha2+\beta2 W2+\gamma2 W2^2+\delta2 W2^3$$

$$AI=\alpha3+\beta3 W3+\gamma3 W3^2+\delta3 W3^3$$

Therefore, the individual layer thickness has been calculated three times, and values MI, AM and AI are obtained from the three ratios of the measurement pulses. In an ideal case, the three values MI, AM and AI should be identical, but in reality they may differ to different biological conditions being relevant for the individual measurements.

The parameters $\alpha$, $\beta$, $\delta$ and y of the empirical polynomials may be estimated by comparison with other layer thickness measurement methods (like CT) or may be determined by a computer fit (for instance a least square fit). Also empirical data may be used to determine these parameters.

It has been recognized by the inventor that the parameters $\alpha$, $\beta$, $\delta$ and y do not differ strongly between men and women, people having different colour skins, etc.

The individual thickness values MI, AM and AI may include an inaccurate thickness value due to individual problems during the individual measurements. Therefore, an arithmetic average

$$d=(MI+AM+AI)/3$$

may be calculated. Then, d may be compared to MI, AM and AI, and if the deviation of an individual one of MI, AM and AI deviates more than 10% from d, this measurement is eliminated, and the average value "d" is calculated only on the basis of the acceptable measurement results.

If all three measurements are acceptable, the LED 250 emits a blue light, if two are acceptable, a green light is emitted, if only one is acceptable, a yellow light is emitted and if none of the measurements is acceptable a red light is emitted by the diode 250.

**Fig. 6** shows a measurement head 600 according to another exemplary embodiment of the invention.

A first single LED 601 located closest to the detector 109 and is used as one light source. LEDs 602a, 602b, 602c are located further away from the detector 109 and emit simultaneously another pulse of light. The remote LEDs 603a to 603f also are arranged on a circular trajectory and emit light simultaneously. The embodiment of Fig. 6 may make it possible to use LEDs of identical power, thereby contributing to a low cost.

In contrast to this, the measurement head 700 shown in **Fig. 7** has four diodes 701 to 704 having different distances from the photodetector 109. The larger the distance between one of the diodes 701 to 704 and the detector 109 is, the higher is the light intensity of the respective diode 701 to 704. Thus, the entire number of used diodes 701 to 704 may be kept very small in the embodiment of Fig. 7.

In **Fig. 8,** an embodiment of a measurement head 800 is shown in which two photodetectors 801 and 802 are used for redundantly measuring pulses emitted by the diodes 803 to 805. By taking this measure, the measurement results of the diodes 801 and 802 may be averaged to further increase the accuracy.

In the embodiment of **Fig. 9,** a measurement head 900 is shown in which a CCD detector 901 is used as a multiple pixel detector for detecting signals from the individual diodes 902, 903 and from the diode groups 904a to 904d and 905a to 905d.

**Fig. 10** shows a diagram 1000 having an abscissa 1001 along which fat layer thicknesses of a proband are plotted which are obtained by a computed tomography. Along an ordinate 1002 corresponding layer thicknesses obtained by the Lipometer according to an exemplary embodiment is plotted. As can be seen from a regression line in the diagram 1000, the correspondence of the measurement is very good.

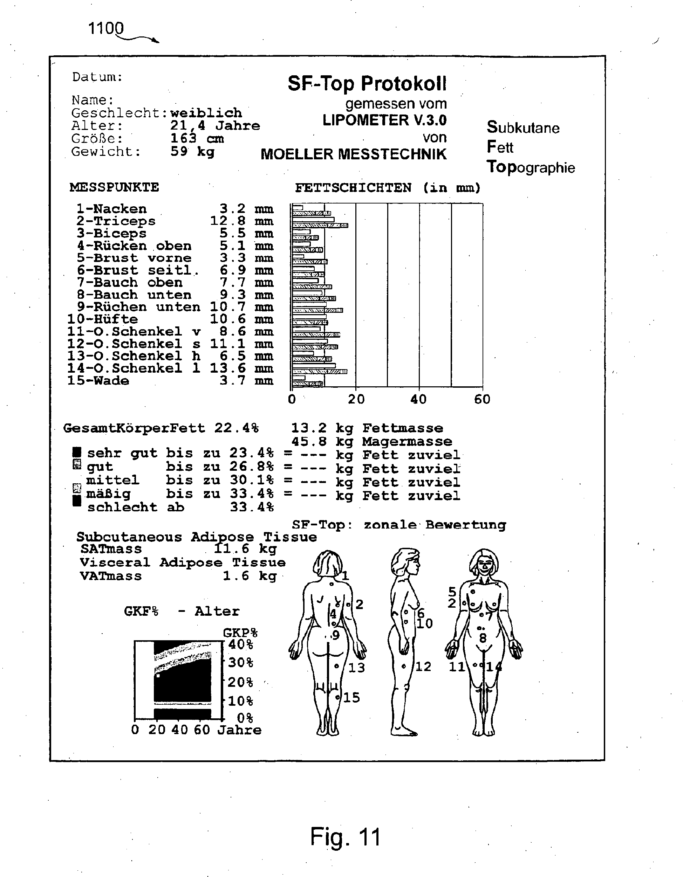

**Fig. 11** shows a protocol 1100 at which the result of individual measurements of the Lipometer according to an exemplary embodiment of the invention is shown, wherein the layer thickness has been measured at 15 different positions at a body of a woman. The results are displayed in an intuitive manner.

**Fig. 12** shows the different positions at which the layer thicknesses have been determined for the woman of Fig. 11. This includes a front chest 1200, a biceps 1201, an upper abdomen 1202, a lower abdomen 1203, a front thigh 1204, an inner thigh 1205, a lateral chest 1206, a hip 1207, a lateral thigh 1208, a neck 1209, an upper back 1210, a triceps 1211, a lower back 1212, a rear thigh 1213, and a calf 1214.

The 15 individual measurement results according to Fig. 11 and Fig. 12 are then plotted in a meaningful diagram 1300 which is shown in **Fig. 13.**

Along an abscissa 1301, the trunk obesity is plotted from the body portions 1200 to 1214 which relate to the trunk of the proband. Along an ordinate 1302, the extremities obesity is plotted wherein only measurement results 1200 to 1214 are used which relate to the extremities. A male diagram 1304 indicative of a typical man at different ages is plotted as well as a corresponding line 1303 for women at different ages. As can be taken from the diagram 1300, specific portions in the landscape diagram 1300 indicate specific risks for diseases, for instance a region 1305 indicates a region where female (f) probands have a high risk of having the disease of diabetes type II (D2).

It should be noted that the term "comprising" does not exclude other elements or features and the "a" or "an" does not exclude a plurality. Also elements described in association with different embodiments may be combined.

It should also be noted that reference signs in the claims shall not be construed as limiting the scope of the claims.

**Claims**

1. An apparatus (100) for determining a thickness of a layer of fat (104) of an organism, the apparatus comprising
   a plurality of electromagnetic radiation sources (101, 102, 103, 601, 62a, 602b, 602c, 603a-603f, 701-704, 803-805, 902, 903, 904a-904d, 905a-905d) adapted for illuminating the layer of fat (104) with a plurality of pulses (107) of electromagnetic radiation;
   at least one electromagnetic radiation detector (109, 801, 802, 901) adapted for detecting detection signals (108, 420, 440, 460) representing the pulses (107) of electromagnetic radiation after transmission through the layer of fat (104);
   a determining unit (110) adapted for determining a plurality of values of the thickness (MI AM, AI) of the layer of fat (104) based on an analysis of a plurality of ratios (W1, W2, W3) between the detection signals (108),
   **characterized in that**
   only those values are used for calculating an average thickness (d) of the layer of fat (104) which values result in deviations from the average thickness (d) of less than a predetermined threshold value; wherein the thickness of the fat layer is calculated a plurality of times and therefore redundantly based on the different ratios of the detection signals and, when a preliminary average value from each of the these individual values has then been calculated, it is verified whether one or more of the calculated individual values deviates more than the predetermined threshold value from the preliminary average value, and, if this is the case, then the individual values which deviate more than the predetermined threshold value from the preliminary average value are neglected for the final determination of the thickness of the fat layer, wherein the preliminary average value is a arithmetic average value or a median of the individual values.

2. The apparatus of claim 1,
   wherein the determining unit (110) is adapted for determining the plurality of values of the thickness (MI AM, AI) of the layer of fat based on a correlation function correlating the plurality of ratios (W1, W2, W3) between the detection signals (108) with the plurality of values of the thickness (MI AM, AI) of the layer (104).

3. The apparatus of claim 2,
   wherein the correlation function is a polynomial function, particularly a polynomial function of third order.

4. The apparatus of any one of claims 1 to 3,
   wherein the determining unit (110) is adapted for calculating the average thickness (d) of the layer of fat (104) using detection signals during the pulses and using detection signals in time intervals between the pulses.

5. The apparatus of any one of claims 1 to 4,
   wherein the determining unit (110) is adapted for performing an underground correction by subtracting an arithmetic average of detection signals in time intervals before and after a pulse from a detection signal during the pulse.

6. The apparatus of any one of claims 1 to 5,
   wherein the plurality of electromagnetic radiation sources (101, 102, 103, 601, 62a, 602b, 602c, 603a-603f, 701-704, 803-805, 902, 903, 904a-904d, 905a-905d) are adapted for illuminating the layer of fat (104) with a plurality of repetitive sequences of the plurality of pulses of electromagnetic radiation; and wherein the determining unit (110) is adapted for adding corresponding detection signals of the plurality of repetitive sequences.

7. The apparatus of any one of claims 1 to 6,
   comprising a quality estimation unit adapted for estimating a quality of the determination of the thickness of the layer of fat based on an amount of values used for calculating the average thickness (d) of the layer of fat (104).

8. The apparatus of claim 7,
   wherein the quality estimation unit is adapted for outputting the estimated quality perceivable for a user.

9. The apparatus of any one of claims 1 to 8,
   wherein the plurality of electromagnetic radiation sources (101, 102, 103, 601, 62a, 602b, 602c, 603a-603f, 701-704, 803-805, 902, 903, 904a-904d, 905a-905d) and the at least one electromagnetic radiation detector (109, 801, 802, 901) are located in a casing in a manner that the plurality of electromagnetic radiation sources and the at least one electromagnetic radiation detector are positionable directly onto a body portion of the organism at which the thickness of the layer of fat (104) is to be determined.

10. The apparatus of any one of claims 1 to 9, wherein the apparatus is adapted for determining the thickness of the layer of fat (104) at a plurality of body portions of the organism and comprises a diagnosis unit adapted for deriving a medical diagnosis regarding at least one illness based on a relationship of the thickness of the layer of fat at the plurality of body portions of the organism.

11. The apparatus of any one of claims 1 to 10, wherein the number of at least one of the plurality of electromagnetic radiation sources (101, 102, 103, 601, 62a, 602b, 602c, 603a-603f, 701-704, 803-805, 902, 903, 904a-904d, 905a-905d) illuminating the layer of fat (104) differs for different ones of the plurality of pulses of electromagnetic radiation, wherein the number is the larger the larger the distance of the respective at least one of the plurality of electromagnetic radiation sources from the at least one electromagnetic radiation detector (109, 801, 802, 901) is.

12. The apparatus of any one of claims 1 to 11, wherein an illumination intensity of at least one of the plurality of electromagnetic radiation sources (101, 102, 103, 601, 62a, 602b, 602c, 603a-603f, 701-704, 803-805, 902, 903, 904a-904d, 905a-905d) illuminating the layer of fat (104) differs for different ones of the plurality of pulses of electromagnetic radiation, wherein the illumination intensity is the larger the larger the distance of the respective at least one of the plurality of electromagnetic radiation sources from the at least one electromagnetic radiation detector (109, 801, 802, 901) is.

13. The apparatus of any one of claims 1 to 12, adapted as a portable device, particularly as one of the group consisting of a mobile phone, a personal digital assistant, an MP3 player, a gaming device, an audio player, a DVD player, a CD player, a harddisk-based media player, a medical communication system, and a body-worn device.

14. A method of determining a thickness of a layer of fat (104) of an organism, the method comprising illuminating the layer of fat (104) with a plurality of pulses (107) of electromagnetic radiation; detecting detection signals (108, 420, 440, 460) representing the pulses (107) of electromagnetic radiation after transmission through the layer of fat (104); determining a plurality of values (MI AM, AI) of the thickness of the layer of fat (104) based on an analysis of a plurality of ratios (W1, W2, W3) between the detection signals,

**characterized in that**

only those values are used for calculating an average thickness (d) of the layer of fat (104) which values result in deviations from the average thickness (d) of less than a predetermined threshold value; wherein the thickness of the fat layer is calculated a plurality of times and therefore redundantly based on the different ratios of the detection signals and, when a preliminary average value from each of the these individual values has then been calculated, it is verified whether one or more of the calculated individual values deviates more than the predetermined threshold value from the preliminary average value of the individual values, and, if this is the case, then the individual values which deviate more than the predetermined threshold value from the preliminary average value are neglected for the final determination of the thickness of the fat layer, wherein the preliminary average value is a arithmetic average value or a median of the individual values.

15. A program element, which, when being executed by a processor, is adapted to control or carry out a method of claim 14 of determining a thickness of a layer of fat (104) of an organism.

16. A computer-readable medium, in which a computer program is stored which, when being executed by a processor, is adapted to control or carry out a method of claim 14 of determining a thickness of a layer of fat (104) of an organism.

**Patentansprüche**

1. Eine Vorrichtung (100) zum Bestimmen einer Dicke von einer Fettschicht (104) von einem Organismus, die Vorrichtung aufweisend
eine Mehrzahl von elektromagnetischen Strahlungsquellen (101, 102, 103, 601, 62a, 602b, 602c, 603a-603f, 701-704, 803-805, 902, 903, 904a-904d, 905a-905d), eingerichtet zum Beleuchten der Fettschicht (104) mit einer Mehrzahl von Pulsen (107) von elektromagnetischer Strahlung;
zumindest einen elektromagnetische Strahlungsdetektor (109, 801, 802, 901), eingerichtet zum Detektieren von Detektiersignalen (108, 420, 440, 460), welche die Pulse (107) von elektromagnetischer Strahlung nach Transmission durch die Fettschicht (104) repräsentieren;
eine bestimmende Einheit (110), eingerichtet zum Bestimmen einer Mehrzahl von Werten von der Dicke (MI, AM, AI) von der Fettschicht (104) basierend auf einer Analyse von einer Mehrzahl von Verhältnissen (W1, W2, W3) zwischen den Detektiersignalen (108),

**dadurch gekennzeichnet, dass**

nur diese Werte zum Berechnen einer Durchschnittsdicke (d) von der Fettschicht (104) verwendet werden, welche Werte zu Abweichungen von der Durchschnittsdicke (d) von weniger als einem vorbestimmten Schwellwert führen; wobei die Dicke von der Fettschicht mehrmals berechnet wird und deswegen redundant basierend auf den verschiedenen Verhältnissen von den Detektiersignalen ist, und wenn ein vorläufiger Durchschnittswert von jedem von diesen individuellen Werten berechnet worden ist, wird verifiziert, ob einer oder mehr von den berechneten individuellen Werten mehr als der vorbestimmte Schwellwert von dem vorläufigen Durchschnittswert abweicht, und, wenn dies der Fall ist, dann werden die individuellen Werte, welche mehr als der vorbestimmte Schwellwert von dem vorläufigen Durchschnittswert abweichen, für die finale Bestimmung von der Dicke von der Fettschicht verworfen, wobei der vorläufige Durchschnittswert ein arithmetischer Durchschnittswert oder ein Median von den individuellen Werten ist.

2. Die Vorrichtung gemäß Anspruch 1, wobei die bestimmende Einheit (110) eingerichtet ist zum Bestimmen der Mehrzahl von Werten von der Dicke (MI, AM, AI) von der Fettschicht basierend auf einer Korrelationsfunktion, welche die Mehrzahl von Verhältnissen (W1, W2, W3) zwischen den Detektiersignalen (108) mit der Mehrzahl von Werten von der Dicke (MI, AM, AI) von der Schicht (104) korreliert.

3. Die Vorrichtung gemäß Anspruch 2, wobei die Korrelationsfunktion eine polynominale Funktion ist, insbesondere eine polynominale Funktion dritter Ordnung.

4. Die Vorrichtung gemäß einem beliebigen von den Ansprüchen 1 bis 3, wobei die bestimmende Einheit (110) eingerichtet ist zum Berechnen der Durchschnittsdicke (d) von der Fettschicht (104) unter Verwenden von Detektiersignalen während der Pulse und unter Verwenden von Detektiersignalen in Zeitintervallen zwischen den Pulsen.

5. Die Vorrichtung gemäß einem beliebigen von den Ansprüchen 1 bis 4, wobei die bestimmende Einheit (110) eingerichtet ist zum Durchführen einer Untergrund Korrektur mittels Subtrahierens eines arithmetischen Durchschnitts von Detektiersignalen in Zeitintervallen vor und nach einem Puls von einem Detektiersignal während des Pulses.

6. Die Vorrichtung gemäß einem beliebigen von den Ansprüchen 1 bis 5, wobei die Mehrzahl von elektromagnetischen Strahlungsquellen (101, 102, 103, 601, 62a, 602b, 602c, 603a-603f, 701-704, 803-805, 902, 903, 904a-904d, 905a-905d) eingerichtet sind zum Beleuchten der Fettschicht (104) mit einer Mehrzahl von wiederholenden Sequenzen von der Mehrzahl von Pulsen von elektromagnetischer Strahlung; und wobei die bestimmende Einheit (110) eingerichtet ist zum Addieren korrespondierender Detektiersignale von der Mehrzahl von wiederholenden Sequenzen.

7. Die Vorrichtung gemäß einem beliebigen von den Ansprüchen 1 bis 6, aufweisend eine Qualitätsabschätz Einheit, eingerichtet zum Abschätzen einer Qualität von der Bestimmung von der Dicke von der Fettschicht basierend auf einer Menge von Werten, welche zum Berechnen der Durchschnittsdicke (d) von der Fettschicht (104) verwendet werden.

8. Die Vorrichtung gemäß Anspruch 7, wobei die Qualitätsabschätz Einheit eingerichtet ist zum, für einen Anwender wahrnehmbaren, Ausgeben der abgeschätzten Qualität.

9. Die Vorrichtung gemäß einem beliebigen von den Ansprüchen 1 bis 8, wobei die Mehrzahl von elektromagnetischen Strahlungsquellen (101, 102, 103, 601, 62a, 602b, 602c, 603a-603f, 701-704, 803-805, 902, 903, 904a-904d, 905a-905d) und der zumindest eine elektromagnetische Strahlungsdetektor (109, 801, 802, 901) in solch einer Weise in einem Gehäuse lokalisiert sind, dass die Mehrzahl von elektromagnetischen Strahlungsquellen und der zumindest eine elektromagnetische Strahlungsdetektor direkt auf einem Körperteil von dem Organismus positionierbar sind, an welchem die Dicke von der Fettschicht (104) zu bestimmen ist.

10. Die Vorrichtung gemäß einem beliebigen von den Ansprüchen 1 bis 9, wobei die Vorrichtung eingerichtet ist zum Bestimmen der Dicke von der Fettschicht (104) an einer Mehrzahl von Körperteilen von dem Organismus und eine Diagnose Einheit aufweist, welche eingerichtet ist zum Ableiten einer medizinischen Diagnose in Bezug auf zumindest eine Krankheit basierend auf einem Zusammenhang von der Dicke von der Fettschicht an der Mehrzahl von Körperteilen von dem Organismus.

11. Die Vorrichtung gemäß einem beliebigen von den Ansprüchen 1 bis 10, wobei die Anzahl von zumindest einer von der Mehrzahl von elektromagnetischen Strahlungsquellen (101, 102, 103, 601, 62a, 602b, 602c, 603a-603f, 701-704, 803-805, 902, 903, 904a-904d, 905a-905d), welche die Fettschicht (104) beleuchten, sich für unterschiedliche von der Mehrzahl von Pulsen von elektromagnetischer Strahlung unterscheidet, wobei die Anzahl umso größer ist, je größer die Distanz von der entsprechenden zumindest einen von der Mehrzahl von elektromagnetischen Strahlungsquellen zu dem zumindest einen elektromagnetischen Strahlungsdetektor (109, 801, 802, 901) ist.

12. Die Vorrichtung gemäß einem beliebigen von den Ansprüchen 1 bis 11,
wobei eine Beleuchtungsintensität von zumindest einer von der Mehrzahl von elektromagnetischen Strahlungsquellen (101, 102, 103, 601, 62a, 602b, 602c, 603a-603f, 701-704, 803-805, 902, 903, 904a-904d, 905a-905d), welche die Fettschicht (104) beleuchten, sich für unterschiedliche von der Mehrzahl von Pulsen von elektromagnetischer Strahlung unterscheiden, wobei die Beleuchtungsintensität umso größer ist, je größer die Distanz von der entsprechenden zumindest einen von der Mehrzahl von elektromagnetischen Strahlungsquellen zu dem zumindest einen elektromagnetischen Strahlungsdetektor (109, 801, 802, 901) ist.

13. Die Vorrichtung gemäß einem beliebigen von den Ansprüchen 1 bis 12,
eingerichtet als ein tragbares Gerät, insbesondere als eines aus der Gruppe, welche besteht aus einem Mobiltelefon, einem persönlichen digitalen Assistenten, einem MP3 Player, einem Spielgerät, einem Audioplayer, einem DVD-Player, einem CD-Player, einem Festplatte-basierten Media Player, einem medizinischen Kommunikationssystem, und einem am Körper getragenen Gerät.

14. Ein Verfahren zum Bestimmen einer Dicke von einer Fettschicht (104) von einem Organismus, das Verfahren aufweisend
Beleuchten der Fettschicht (104) mit einer Mehrzahl von Pulsen (107) von elektromagnetischer Strahlung;
Detektieren von Detektiersignalen (108, 420, 440, 460), welche die Pulse (107) von elektromagnetischer Strahlung nach Transmission durch die Fettschicht (104) repräsentieren;
Bestimmen einer Mehrzahl von Werten (MI, AM, AI) von der Dicke der Fettschicht (104) basierend auf einer Analyse von einer Mehrzahl von Verhältnissen (W1, W2, W3) zwischen den Detektiersignalen,
**dadurch gekennzeichnet, dass**
nur diese Werte zum Berechnen einer Durchschnittsdicke (d) von der Fettschicht (104) verwendet werden, welche Werte zu Abweichungen von der Durchschnittsdicke (d) von weniger als einem vorbestimmten Schwellwert führen; wobei die Dicke von der Fettschicht mehrmals berechnet ist und deswegen redundant basierend auf den verschiedenen Verhältnissen von den Detektiersignalen ist, und wenn ein vorläufiger Durchschnittswert von jedem von diesen individuellen Werten berechnet worden ist, wird verifiziert, ob einer oder mehr von den berechneten individuellen Werten mehr als der vorbestimmte Schwellwert von dem vorläufigen Durchschnittswert der individuellen Werte abweicht, und, wenn dies der Fall ist, dann werden die individuellen Werte, welche mehr als der vorbestimmte Schwellwert von dem vorläufigen Durchschnittswert abweichen, für die finale Bestimmung von der Dicke von der Fettschicht verworfen,
wobei der vorläufige Durchschnittswert ein arithmetischer Durchschnittswert oder ein Median von den individuellen Werten ist.

15. Ein Programmelement, welches, wenn es mittels eines Prozessors ausgeführt wird, eingerichtet ist zum Steuern oder Durchführen eines Verfahrens gemäß Anspruch 14 zum Bestimmen einer Dicke von einer Fettschicht (104) von einem Organismus.

16. Ein Computer-lesbares Medium, in welchem ein Computerprogramm gespeichert ist, welches, wenn es mittels eines Prozessors ausgeführt wird, eingerichtet ist zum Steuern oder Durchführen eines Verfahrens gemäß Anspruch 14 zum Bestimmen einer Dicke von einer Fettschicht (104) von einem Organismus.

**Revendications**

1. Appareil (100) pour déterminer une épaisseur d'une couche de graisse (104) d'un organisme, l'appareil comprenant
une pluralité de 'sources de rayonnement électromagnétique (101, 102, 103, 601, 62a, 602b, 602c, 603a à 603f, 701 à 704, 803 à 805, 902, 903, 904a à 904d, 905a à 905d) adaptées pour illuminer la couche de graisse (104) avec une pluralité d'impulsions (107) de rayonnement électromagnétique ;
au moins un détecteur de rayonnement électromagnétique (109, 801, 802, 901) adapté pour détecter des signaux de détection (108, 420, 460) représentant les impulsions (107) de rayonnement électromagnétique après transmission à travers la couche de graisse (104) ;
une unité de détermination (110) adaptée pour déterminer une pluralité de valeurs de l'épaisseur (MI AM, AI) de la couche de graisse (104) en se basant sur une analyse d'une pluralité de rapports (W1, W2, W3) entre les signaux de détection (108),
**caractérisé en ce que** :

on n'utilise pour calculer une épaisseur moyenne (d) de la couche de graisse (104) uniquement les valeurs qui conduisent à des écarts par rapport à l'épaisseur moyenne (d) de moins d'une valeur seuil prédéterminée ; dans lequel l'épaisseur de la couche de graisse est calculée une pluralité de fois et en conséquence de façon redondante en se basant sur les différents rapports des signaux de détection et, lorsqu'une valeur moyenne préliminaire de chacune de ces valeurs individuelles a alors été calculée, on vérifie si une ou plusieurs des valeurs individuelles calculées s'écarte de plus de la valeur seuil prédéterminée par rapport à la valeur moyenne préliminaire, et, si c'est le cas, alors les valeurs individuelles qui s'écartent de plus de la valeur seuil prédéterminée par rapport à la valeur moyenne préliminaire sont négligées pour la détermination finale de l'épaisseur de la couche de graisse, dans lequel la valeur moyenne préliminaire est une valeur moyenne arithmétique ou une médiane des valeurs individuelles.

**2.** Appareil selon la revendication 1,
dans lequel l'unité de détermination (110) est adaptée pour déterminer la pluralité des valeurs de l'épaisseur (MI AM, AI) de la couche de graisse en se basant sur une fonction de corrélation corrélant la pluralité de rapports (W1, W2, W3) entre les signaux de détection (108) avec la pluralité de valeurs de l'épaisseur (MI AM, AI) de la couche (104).

**3.** Appareil selon la revendication 2,
dans lequel la fonction de corrélation est une fonction polynomiale, en particulier une fonction polynomiale de troisième ordre.

**4.** Appareil selon l'une quelconque des revendications 1 à 3,
dans lequel l'unité de détermination (110) est adaptée pour calculer l'épaisseur moyenne (d) de la couche de graisse (104) en utilisant des signaux de détection pendant les impulsions et en utilisant des signaux de détection en intervalles de temps entre les impulsions.

**5.** Appareil selon l'une quelconque des revendications 1 à 4,
dans lequel l'unité de détermination (110) est adaptée pour réaliser une correction de fond en soustrayant une moyenne arithmétique de signaux de détection en intervalles de temps avant et après une impulsion à partir d'un signal de détection pendant l'impulsion.

**6.** Appareil selon l'une quelconque des revendications 1 à 5,
dans lequel la pluralité de sources de rayonnement électromagnétique (101, 102, 103, 601, 62a, 602b, 602c, 603a à 603f, 701 à 704, 803 à 805, 902, 903, 904a à 904d, 905a à 905d) sont adaptées pour illuminer la couche de graisse (104) avec une pluralité de séquences répétitives de la pluralité d'impulsions de rayonnement électromagnétique ; et
dans lequel l'unité de détermination (110) est adaptée pour additionner des signaux de détection correspondants de la pluralité de séquences répétitives.

**7.** Appareil selon l'une quelconque des revendications 1 à 6,
comprenant une unité d'estimation de qualité adaptée pour estimer une qualité de la détermination de l'épaisseur de la couche de graisse en se basant sur une quantité de valeurs utilisées pour calculer l'épaisseur moyenne (d) de la couche de graisse (104).

**8.** Appareil selon la revendication 7,
dans lequel l'unité d'estimation de qualité est adaptée pour produire en sortie la qualité estimée percevable pour un utilisateur.

**9.** Appareil selon l'une quelconque des revendications 1 à 8,
dans lequel la pluralité de sources de rayonnement électromagnétique (101, 102, 103, 601, 62a, 602b, 602c, 603a à 603f, 701 à 704, 803 à 805, 902, 903, 904a à 904d, 905a à 905d) et le au moins un détecteur de rayonnement électromagnétique (109, 801, 802, 901) sont situés dans un boîtier de manière à ce que la pluralité de sources de rayonnement électromagnétique et le au moins un détecteur de rayonnement électromagnétique soient positionnables directement sur une portion du corps de l'organisme au niveau duquel l'épaisseur de la couche de graisse (104) est à déterminer.

**10.** Appareil selon l'une quelconque des revendications 1 à 9,
dans lequel l'appareil est adapté pour déterminer l'épaisseur de la couche de graisse (104) en une pluralité de portions du corps de l'organisme et comprend une unité de diagnostic adaptée pour dériver un diagnostic médical concernant au moins une maladie en se basant sur une relation de l'épaisseur de la couche de graisse au niveau de la pluralité de portions du corps de l'organisme.

**11.** Appareil selon l'une quelconque des revendications 1 à 10,
dans lequel le nombre d'au moins l'une de la pluralité de sources de rayonnement électromagnétique (101, 102, 103, 601, 62a, 602b, 602c, 603a à 603f, 701 à 704, 803 à 805, 902, 903, 904a à 904d, 905a à 905d) illuminant la couche de graisse (104) diffère pour des impulsions différentes de la pluralité d'impulsions de rayonnement électromagnétique, dans lequel le nombre est d'autant plus grand que la distance de la au moins une respective de la pluralité de sources de rayonnement électromagnétique par rapport au au moins un détecteur de rayonnement électromagnétique (109, 801, 802, 901) est grande.

12. Appareil selon l'une quelconque des revendications 1 à 11,
dans lequel une intensité d'illumination d'au moins l'une de la pluralité de sources de rayonnement électromagnétique (101, 102, 103, 601, 62a, 602b, 602c, 603a à 603f, 701 à 704, 803 à 805, 902, 903, 904a à 904d, 905a à 905d) illuminant la couche de graisse (104) diffère pour des impulsions différentes de la pluralité d'impulsions de rayonnement électromagnétique, dans lequel l'intensité d'illumination est d'autant plus grande que la distance de la au moins une respective de la pluralité de sources de rayonnement électromagnétique par rapport au au moins un détecteur de rayonnement électromagnétique (109, 801, 802, 901) est grande.

13. Appareil selon l'une quelconque des revendications 1 à 12,
adapté en tant que dispositif portable, en particulier en tant qu'un élément du groupe consistant en un téléphone mobile, un assistant numérique personnel, un lecteur MP3, un dispositif de jeu, un lecteur audio, un lecteur de DVD, un lecteur de CD, un lecteur de support à base de disque dur, un système de communication médical et un dispositif porté sur le corps.

14. Procédé de détermination d'une épaisseur d'une couche de graisse (104) d'un organisme, le procédé comprenant :

l'illumination de la couche de graisse (104) avec une pluralité d'impulsions (107) de rayonnement électromagnétique ;
la détection de signaux de détection (108, 420, 440, 460) représentant les impulsions (107) de rayonnement électromagnétique après transmission à travers la couche de graisse (104) ;
la détermination d'une pluralité de valeurs (MI AM, AI) de l'épaisseur de la couche de graisse (104) en se basant sur une analyse d'une pluralité de rapports (W1, W2, W3) entre les signaux de détection,
**caractérisé en ce que**
on n'utilise pour calculer une épaisseur moyenne (d) de la couche de graisse (104) uniquement les valeurs qui conduisent à des écarts par rapport à l'épaisseur moyenne (d) de moins d'une valeur seuil prédéterminée ; dans lequel l'épaisseur de la couche de graisse est calculée une pluralité de fois et en conséquence de façon redondante en se basant sur les différents rapports des signaux de détection et, lorsqu'une valeur moyenne préliminaire de chacune de ces valeurs individuelles a alors été calculée, on vérifie si une ou plusieurs des valeurs individuelles calculées s'écarte de plus de la valeur seuil prédéterminée par rapport à la valeur moyenne préliminaire, et, si c'est le cas, alors les valeurs individuelles qui s'écartent de plus de la valeur seuil prédéterminée par rapport à la valeur moyenne préliminaire sont négligées pour la détermination finale de l'épaisseur de la couche de graisse, dans lequel la valeur moyenne préliminaire est une valeur moyenne arithmétique ou une médiane des valeurs individuelles.

15. Elément de programme qui, lorsqu'il est exécuté par un processeur, est adapté pour commander ou réaliser un procédé de la revendication 14 de détermination d'une épaisseur d'une couche de graisse (104) d'un organisme.

16. Support lisible par ordinateur, dans lequel un programme informatique est stocké, lequel, lorsqu'il est exécuté par un processeur, est adapté pour commander ou réaliser un procédé de la revendication 14 de détermination d'une épaisseur d'une couche de graisse (104) d'un organisme.

**Fig. 1**

**Fig. 2A**

**Fig. 2B**

**Fig. 3**

Fig. 4

Fig. 5

Fig. 6

Fig. 7

Fig. 8

Fig. 9

Fig. 10

Fig. 12

Fig. 13

1100

| | |
|---|---|
| Datum: | **SF-Top Protokoll** |
| Name: | gemessen vom |
| Geschlecht: weiblich | **LIPOMETER V.3.0** |
| Alter: 21,4 Jahre | von |
| Größe: 163 cm | **MOELLER MESSTECHNIK** |
| Gewicht: 59 kg | |

**Subkutane Fett Topographie**

**MESSPUNKTE**     **FETTSCHICHTEN** (in mm)

```
 1-Nacken           3.2 mm
 2-Triceps         12.8 mm
 3-Biceps           5.5 mm
 4-Rücken oben      5.1 mm
 5-Brust vorne      3.3 mm
 6-Brust seitl.     6.9 mm
 7-Bauch oben       7.7 mm
 8-Bauch unten      9.3 mm
 9-Rüchen unten    10.7 mm
10-Hüfte           10.6 mm
11-O.Schenkel v     8.6 mm
12-O.Schenkel s    11.1 mm
13-O.Schenkel h     6.5 mm
14-O.Schenkel l    13.6 mm
15-Wade             3.7 mm
```

0   20   40   60

GesamtKörperFett 22.4%     13.2 kg Fettmasse
                           45.8 kg Magermasse
■ sehr gut bis zu 23.4% = --- kg Fett zuviel
▩ gut     bis zu 26.8% = --- kg Fett zuviel
  mittel  bis zu 30.1% = --- kg Fett zuviel
▨ mäßig   bis zu 33.4% = --- kg Fett zuviel
■ schlecht ab    33.4%

SF-Top: zonale Bewertung

Subcutaneous Adipose Tissue
  SATmass          11.6 kg
Visceral Adipose Tissue
  VATmass           1.6 kg

GKF%  - Alter

GKP%
40%
30%
20%
10%
0%
0 20 40 60 Jahre

**Fig. 11**

**EP 2 091 415 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5014713 A
- US 2007185399 A
- EP 0516251 A
- US 20050043598 A1
- AT 201095